# EUROPÄISCHE PATENTSCHRIFT

(11) **EP 2 547 250 B1**
(45) Veröffentlichungstag und Bekanntmachung des Hinweises auf die Patenterteilung: **27.05.2015**
(21) Anmeldenummer: 11708742.9
(22) Anmeldetag: 15.03.2011
(51) Int. Cl.: A61B 5/00

(54) **BIODETEKTOR**
BIODETECTOR
BIODÉTECTEUR

(30) Priorität: 16.03.2010 DE 102010011560
(43) Veröffentlichungstag der Anmeldung: 23.01.2013
(73) Patentinhaber: GILUPI GmbH, 14476 Potsdam - Golm (DE)
(72) Erfinder: WEBER, Ekkehardt, 06124 Halle (Saale) (DE); NIESTROJ, Robert, 10249 Berlin (DE)
(74) Vertreter: Grünecker Patent- und Rechtsanwälte PartG mbB
(86) Internationale Anmeldenummer: PCT/EP2011/001293
(87) Internationale Veröffentlichungsnummer: WO 2011/113584

(56) Entgegenhaltungen:
- EP-A1- 1 800 751
- WO-A1-01/21064
- WO-A1-2006/131400
- WO-A2-91/06855
- WO-A2-2005/024397
- DE-A1- 3 025 800

## Beschreibung

Die Erfindung betrifft einen Biodetektor mit einer funktionalisierten Fläche zur Isolierung von Molekülen oder Zellen aus dem menschlichen Körper.

Ein derartiger Biodetektor ist beispielsweise aus der WO 2006/131400 A1 bekannt. Dieser Biodetektor wird zur Isolierung und Anreicherung von Zielmolekülen und Zielzellen in den menschlichen Körper eingebracht und nach kurzer Verweildauer wieder aus dem menschlichen Körper entnommen. Beim Einbringen des Biodetektors in den Körper oder bei der Entnahme des Biodetektors aus dem Körper wird die funktionalisierte Fläche bzw. darauf angereichertes Material leicht abgerieben. Ferner ist zu gewährleisten, dass die funktionalisierte Fläche des Biodetektors biokompatibel ist und möglichst keine Abwehrreaktionen auslöst.

Der Erfindung liegt die Aufgabe zugrunde, einen Biodetektor der eingangs genannten Art derart zu verbessern, dass die funktionalisierte Fläche des Biodetektors bzw. darauf angereicherte Moleküle oder Zellen einem geringeren Abrieb ausgesetzt sind, und die Biokompatibilität des Biodetektors verbessert wird.

Um die der Erfindung zugrunde liegende Aufgabe zu lösen, stellt die Erfindung den Biodetektor mit einer funktionalisierten Fläche zur Isolierung von Molekülen oder Zellen aus dem menschlichen Körper nach Anspruch 1 bereit, welcher derart ausgebildet ist, um eine Flüssigkeit aus dem menschlichen Körper zu entnehmen und in einem Innenraum des Biodetektors aufzunehmen, wobei die funktionalisierte Fläche dem Innenraum des Biodetektors zugewandt ist. Durch den erfindungsgemäßen Biodetektor kann eine bestimmte Menge einer Körperflüssigkeit entnommen und im Biodetektor aufgenommen sowie ggf. analysiert werden. Die funktionalisierte Fläche sowie die daran angereichten Zielmoleküle oder Zielzellen sind im Innenraum des Biodetektors vor Abrieb geschützt. Ferner wird eine mit der funktionalisierten Fläche in Kontakt gelangende Körperflüssigkeit bestimmungsgemäß nicht wieder in den Körper zurückgeführt und ist nicht mehr Bestandteil einer im Körper zirkulierenden Flüssigkeitsmenge. Somit ist die Verwendung von humanen Bestandteilen auf der funktionalisierten Fläche nicht mehr erforderlich und sämtliche Biokompatibilitätstests können entfallen.

Bevorzugte Weiterbildungen der Erfindung sind Gegenstände der Unteransprüche

Es kann sich als vorteilhaft erweisen, wenn die funktionalisierte Fläche zumindest abschnittsweise mit Detektionsmolekülen bzw. einem Rezeptor oder Liganden besetzt ist. Dadurch können ausgewählte Zielmoleküle und Zielzellen einfach angereichert werden.

Es wird weiter als vorteilhaft angesehen, wenn als Detektionsmoleküle Antikörper, vorzugsweise monoklonale Antikörper, chimäre Antikörper, humanisierte Antikörper, Fragmente von Antikörpern oder Aminosäure-Strukturen bzw. Aminosäure-Sequenzen, Nukleinsäure-Strukturen bzw. Nukleinsäure-Sequenzen, Kohlehydratstrukturen oder synthetische Strukturen eingesetzt werden.

Es kann aber auch nützlich sein, wenn die Detektionsmoleküle nicht-humanen Ursprungs, vorzugsweise tierischen Ursprungs, bevorzugt murinen Ursprungs, sind. Derartige Detektionsmoleküle ermöglichen ein größeres Spektrum zur Anreicherung von Zielmolekülen und Zielzellen als humane Detektionsmoleküle alleine.

Es kann sich als hilfreich erweisen, wenn die Detektionsmoleküle, vorzugsweise mittels Protein G, orientiert an der funktionalisierten Fläche angebunden sind. Protein G weist für bestimmte Immunglobuline eine hohe Affinität auf.

Es wird aber auch als praktisch angesehen, wenn die Detektionsmoleküle kovalent an der funktionalisierten Fläche angebunden sind.

In einer vorteilhaften Weiterbildung der Erfindung weist der Biodetektor einen Flüssigkeitsentnahmeabschnitt auf, der zumindest eine der folgenden Anforderungen erfüllt:
- Der Flüssigkeitsentnahmeabschnitt umfasst eine Öffnung, über welche die Flüssigkeit in den Innenraum des Biodetektors gelangt. Dadurch kann die Aufnahme der Flüssigkeit gut kontrolliert werden.
- Ein Innenraum des Flüssigkeitsentnahmeabschnitts kommuniziert mit der Öffnung. Dadurch kann der Flüssigkeitsentnahmeabschnitt bereits selbst eine bestimmte Flüssigkeitsmenge aufnehmen.
- Die Öffnung ist an einem Ende des Flüssigkeitsentnahmeabschnitts ausgebildet. Dadurch wird die aufgenommene Flüssigkeitsmenge nur in einer Richtung durch den Flüssigkeitsentnahmeabschnitt geführt. Die Gefahr eines unkontrollierten Rückfließens der aufgenommenen Flüssigkeit kann dadurch weitgehend verringert werden.
- Der Flüssigkeitsentnahmeabschnitt ist zumindest abschnittsweise aus einem biokompatiblen Material ausgebildet. Dadurch kann eine Abwehrreaktion des menschlichen Körpers weitgehend verhindert werden.
- Der Flüssigkeitsentnahmeabschnitt ist zumindest abschnittsweise aus Kunststoff, Metall oder Glas, vorzugsweise Edelstahl, Titan oder Glasfaser, bevorzugt einem bioverträglichen Kunststoff oder einer Kombination aus diesen Substanzen, ausgebildet. Derartige Materialien sind leicht formbar.
- Der Flüssigkeitsentnahmeabschnitt ist als Kanüle ausgebildet. Kanülen sind standardisiert in verschiedenen Größen erhältlich. Durch Verwendung von standardisierten Komponenten verringern sich die Herstellungskosten des Biodetektors.
- Der Flüssigkeitsentnahmeabschnitt umfasst einen Außendurchmesser von 0,25 bis 3,5 mm, vorzugsweise 0,5 bis 3,0 mm, weiter vorzugsweise 0,75 bis 2,5 mm, bevorzugt 1,0 mm bis 2,0 mm. Derartige Kanülengrößen sind besonders gängig.
- Die Öffnung umfasst einen Durchmesser von 0,2 bis 3,0 mm, vorzugsweise 0,25 bis 2,5 mm, weiter vorzugsweise 0,5 bis 2,0 mm, bevorzugt 0,75 mm bis 1,5 mm. Durch eine Öffnung dieser Größe kann die aufgenommene Flüssigkeitsmenge gut kontrolliert werden.
- Die funktionalisierte Fläche befindet sich an einer Innenwand des Flüssigkeitsentnahmeabschnitts. Dadurch kann bereits der Innenraum des Flüssigkeitsentnahmeabschnitts zur Anreicherung der Zielmoleküle oder Zielzellen genutzt werden. Im Ergebnis kann eine zur Isolierung von Molekülen oder Zellen aus dem menschlichen Körper zu entnehmende Flüssigkeitsmenge verringert werden.
- Der Flüssigkeitsentnahmeabschnitt besteht zumindest abschnittsweise aus einem Material, welches funktionelle Gruppen zur kovalenten Anbindung der Detektionsmoleküle enthält, und/oder welches chemisch oder enzymatisch spaltbare Gruppen enthält, um die quantitative Gewinnung von gebundenen Zielmolekülen oder Zielzellen zu vereinfachen, und/oder welches eine Matrix bildet, welche die Anbindung unspezifischer Zellen oder Wechselwirkungen mit Körperflüssigkeiten verhindert. Dadurch können die Detektionsmoleküle unmittelbar angebunden werden oder die Anreicherung sowie Ablösung der Zielmoleküle oder Zielzellen von der funktionalisierten Fläche vereinfacht werden.

In einer vorteilhaften Weiterbildung der Erfindung weist der Biodetektor einen Leitungsabschnitt auf, der zumindest eine der folgenden Anforderungen erfüllt:
- Ein Innenraum des Leitungsabschnitts kommuniziert mit der Öffnung und/oder mit dem Innenraum des Flüssigkeitsentnahmeabschnitts. Somit kann auch der Innenraum des Leitungsabschnitts eine Flüssigkeitsmenge aufnehmen.
- Der Leitungsabschnitt ist mit dem Flüssigkeitsentnahmeabschnitt verbunden, vorzugsweise lösbar verbunden. Durch den modularen Aufbau können die Bestandteile des Biodetektors leicht ausgetauscht werden. Insbesondere kann der Flüssigkeitsentnahmeabschnitt oder Leitungsabschnitt leicht ausgetauscht werden, was aus hygienischen Gründen vorteilhaft ist. Vorzugsweise umfassen die zu verbindenden Teile standardisierte Verbindungsstücke für medizinische Anwendungen.
- Der Leitungsabschnitt ist als flexibler Schlauch ausgebildet. Dadurch kann der Leitungsabschnitt leicht gebogen werden, was für bestimmte Anwendungen komfortabel ist.
- Der Leitungsabschnitt weist einen größeren Innendurchmesser und/oder Außendurchmesser auf als der Flüssigkeitsentnahmeabschnitt. Durch können der Leitungsabschnitt und der Flüssigkeitsentnahmeabschnitt leicht ineinander gesteckt werden, wobei der Leitungsabschnitt vorzugsweise auf den Flüssigkeitsentnahmeabschnitt aufgesteckt oder aufgeschoben wird und durch alleine durch Reibungskräfte bzw. elastische Rückstellkräfte auf dem Flüssigkeitsentnahmeabschnitt hält. Komplizierte Verbindungsmechanismen können entfallen.
- Der Leitungsabschnitt umfasst einen Innendurchmesser von 0,25 bis 3,5 mm, vorzugsweise 0,5 bis 3,0 mm, weiter vorzugsweise 0,75 bis 2,5 mm, bevorzugt 1,0 mm bis 2,0 mm. Bei diesem Innendurchmesser ist das Verhältnis der mit der funktionalisierten Fläche in Kontakt gelangenden Flüssigkeitsmenge zu der im Innenraum aufgenommenen Flüssigkeitsmenge besonders vorteilhaft.
- Der Leitungsabschnitt umfasst wenigstens eine Verzweigung. Durch eine Verzweigung kann das Verhältnis der mit der funktionalisierten Fläche in Kontakt gelangenden Flüssigkeitsmenge zu der im Innenraum aufgenommenen Flüssigkeitsmenge weiter erhöht werden.
- Der Leitungsabschnitt umfasst wenigstens eine offene Zweigleitung und/oder wenigstens eine kurzgeschlossene Zweigleitung und/oder wenigstens eine verzweigte Zweigleitung. Die offene Zweigleitung verfügt über ein totes Ende und wird kaum oder nur mit geringer Geschwindigkeit durchströmt. Die kurzgeschlossene Zweigleitung ist an beiden Enden mit einer anderen Leitung verbunden und wird mit höherer Geschwindigkeit durchströmt als die offene Zweigleitung. Je nach Anwendung kann eine offene oder kurzgeschlossene Zweigleitung sinnvoll sein. Natürlich kann auch eine Zweigleitung wieder eine oder mehrere Verzweigungen aufweisen, so dass ein beliebig komplexes Leitungssystem erzeugt werden kann.
- Der Leitungsabschnitt umfasst wenigstens drei Zweigleitungen, die sich in unterschiedlichen Ebenen erstrecken. Dadurch können die Zweigleitungen besonders kompakt angeordnet werden.
- Der Leitungsabschnitt umfasst wenigstens eine Querschnittsänderung, vorzugsweise wenigstens eine Querschnittserweiterung und/oder wenigstens eine Querschnittsverringerung. An einer Querschnittsänderung ändern sich die Strömungsverhältnisse, insbesondere die Strömungsgeschwindigkeit der im Leitungsabschnitt aufgenommenen Flüssigkeit. Gerade dort kann es sinnvoll sein, eine funktionalisierte Fläche vorzusehen.
- Die funktionalisierte Fläche befindet sich an einer Innenwand des Leitungsabschnitts. Dadurch ist der Innenraum des Leitungsabschnitts zur Anreicherung der Zielmoleküle oder Zielzellen nutzbar.
- Der Leitungsabschnitt ist als aus Kunststoff, vorzugsweise einem Polymer, bevorzugt Polystyren, ausgebildet. Derartige Materialien weisen vorteilhafte Eigenschaften für Anbindung von Detektionsmolekülen auf. An Polystyren können Antikörper als Detektionsmoleküle adsorbiert werden. Es besteht eine große Auswahl an Polymeren für eine optimale Linkerchemie.
- Der Leitungsabschnitt besteht zumindest abschnittsweise aus einem Material, welches funktionelle Gruppen zur kovalenten Anbindung der Detektionsmoleküle enthält, und/oder welches chemisch oder enzymatisch spaltbare Gruppen enthält, um die quantitative Gewinnung von gebundenen Zielmolekülen oder Zielzellen zu vereinfachen, und/oder welches eine Matrix bildet, welche die Anbindung unspezifischer Zellen oder Wechselwirkungen mit Körperflüssigkeiten verhindert. Dadurch können die Detektionsmoleküle unmittelbar angebunden werden oder die Anreicherung sowie Ablösung der Zielmoleküle oder Zielzellen vereinfacht werden.

In einer anderen vorteilhaften Weiterbildung der Erfindung weist der Biodetektor eine Speichervorrichtung auf, die zumindest eine der folgenden Anforderungen erfüllt:
- Die Speichervorrichtung umfasst ein variables Volumen. Dadurch kann die im Biodetektor aufgenommene Flüssigkeitsmenge fein eingestellt werden.
- Der Innenraum der Speichervorrichtung kommuniziert mit der Öffnung und/oder mit dem Innenraum des Flüssigkeitsentnahmeabschnitts und/oder dem Innenraum des Leitungsabschnitts. Durch Vergrößerung des Speichervolumens kann der gesamte Innenraum des Biodetektors mit der Flüssigkeit befüllt werden. Durch Verringerung des Speichervolumens kann der Innenraum des Biodetektors zumindest teilweise wieder entleert werden.
- Die Speichervorrichtung ist mit dem Leitungsabschnitt verbunden, vorzugsweise lösbar verbunden. Der modulare Aufbau des Biodetektors gemäß dieser Ausführung erleichtert den Austausch einzelner Komponenten für verschiedene Anwendungen.
- Die Speichervorrichtung ist als Spritze ausgebildet. Der Einsatz standardisierter Komponenten gemäß dieser Ausführung verringert die Herstellungskosten des Biodetektors.
- Die funktionalisierte Fläche befindet sich an einer Innenwand der Speichervorrichtung. Dadurch ist der Innenraum der Speichervorrichtung zur Anreicherung der Zielmoleküle oder Zielzellen nutzbar.
- Die Speichervorrichtung besteht zumindest abschnittsweise aus einem Material, welches funktionelle Gruppen zur kovalenten Anbindung der Detektionsmoleküle enthält, und/oder welches chemisch oder enzymatisch spaltbare Gruppen enthält, um die quantitative Gewinnung von gebundenen Zielmolekülen oder Zielzellen zu vereinfachen, und/oder welches eine Matrix bildet, welche die Anbindung unspezifischer Zellen oder Wechselwirkungen mit Körperflüssigkeiten verhindert. Dadurch können die Detektionsmoleküle unmittelbar an der funktionalisierten Fläche der Speichervorrichtung angebunden werden oder die Anreicherung sowie Ablösung der Zielmoleküle oder Zielzellen vereinfacht werden.

In noch einer anderen vorteilhaften Weiterbildung der Erfindung weist der Biodetektor eine Detektionsvorrichtung auf, die zumindest eine der folgenden Anforderungen erfüllt:
- Die Detektionsvorrichtung ist als funktionalisierter Chip ausgebildet. Dadurch kann bereits innerhalb des Biosensors eine genauere Analyse, insbesondere eine computergestützten Erkennung und/oder Bestimmung der angereicherten Zielmoleküle oder Zielzellen stattfinden, bspw. im Hinblick auf die Menge der angereicherten Zielmoleküle oder Zielzellen pro Menge der aufgenommenen Flüssigkeit oder dergleichen. Vorzugsweise weist die Detektionsvorrichtung eine Schnittstelle zur Datenübertragung auf.
- Die Detektionsvorrichtung dient der optischen, vorzugsweise mikroskopisch gestützten Erkennung und/oder Bestimmung der Zielmoleküle oder Zielzellen. In diesem Fall ist vorzugsweise zumindest ein Teil der Detektionsvorrichtung, der mit einer funktionalisierten Fläche versehen ist, transparent oder transluzent ausgebildet. Dieser Teil der Detektionsvorrichtung kann vorzugsweise in eine optische Erkennungseinrichtung, beispielsweise ein Mikroskop, derart eingegeben werden, dass die Zielmoleküle oder Zielzellen, die ggf. in der Detektionsvorrichtung an der funktionalisierten Fläche angeordnet sind, durch die optische Erkennungseinrichtung erkannt werden können.
- Die Detektionsvorrichtung dient der computergestützten Ermittlung und/oder Bestimmung der Zielmoleküle oder Zielzellen. Dadurch kann der Aufwand zur Ermittlung und/oder Bestimmung der Zielmoleküle oder Zielzellen (z. B. Anzahl der Zielmoleküle oder Zielzellen pro Flüssigkeitsmenge) erheblich verringert werden.
- Die Detektionsvorrichtung ist in den Flüssigkeitsentnahmeabschnitt und/oder in den Leitungsabschnitt und/oder in die Speichervorrichtung integriert. Vorzugsweise ist die Detektionsvorrichtung in den Leitungsabschnitt integriert. Dadurch kann die Detektionsvorrichtung leicht mit standardisierten Bauteilen wie Kanüle und Spritze gekoppelt werden.
- Der Innenraum der Detektionsvorrichtung kommuniziert mit der Öffnung und/oder mit dem Innenraum des Flüssigkeitsentnahmeabschnitts und/oder mit dem Innenraum des Leitungsabschnitts und/oder mit dem Innenraum der Speichervorrichtung. Dadurch kann die Detektionsvorrichtung direkt und ohne Umfüllung mit der aus dem Körper entnommenen Flüssigkeit befüllt werden.
- Die Detektionsvorrichtung ist mit dem Flüssigkeitsentnahmeabschnitt und/oder mit dem Leitungsabschnitt und/oder mit der Speichervorrichtung verbunden, vorzugsweise lösbar verbunden. Der modulare Aufbau des Biodetektors gemäß dieser Ausführung erleichtert den Austausch einzelner Komponenten für verschiedene Anwendungen. Ferner kann die Detektionsvorrichtung zur Erkennung und/oder Bestimmung der Zielmoleküle oder Zielzellen einfach von den anderen Komponenten des Biodetektors gelöst werden.
- Die funktionalisierte Fläche befindet sich an einer Innenwand der Detektionsvorrichtung. Dadurch können die Zielmoleküle oder Zielzellen in der Detektionsvorrichtung zugleich angereichert und analysiert werden. Eine Trennung der Zielmoleküle oder Zielzellen von den Detektionsmolekülen zum Zweck der Analyse ist somit nicht erforderlich.
- Die Detektionsvorrichtung besteht zumindest abschnittsweise aus einem Material, welches funktionelle Gruppen zur kovalenten Anbindung der Detektionsmoleküle enthält, und/oder welches chemisch oder enzymatisch spaltbare Gruppen enthält, um die quantitative Gewinnung von gebundenen Zielmolekülen oder Zielzellen zu vereinfachen, und/oder welches eine Matrix bildet, welche die Anbindung unspezifischer Zellen oder Wechselwirkungen mit Körperflüssigkeiten verhindert. Dadurch können die Detektionsmoleküle unmittelbar an der funktionalisierten Fläche der Detektionsvorrichtung angebunden werden oder die Anreicherung sowie Ablösung der Zielmoleküle oder Zielzellen vereinfacht werden.

In noch einer anderen vorteilhaften Weiterbildung der Erfindung weist der Biodetektor eine Sekundärschicht auf, die zumindest eine der folgenden Anforderungen erfüllt:
- Die Sekundärschicht ist als Polymerschicht, vorzugsweise als Hydrogelschicht ausgebildet. Die Polymerschicht erleichtert die Anbindung der Detektionsmoleküle. Durch eine Hydrogelschicht kann eine zur Anreicherung der Zielmoleküle oder Zielzellen funktionalisierte Oberfläche erheblich vergrößert werden.
- Die Sekundärschicht enthält funktionelle Gruppen zur kovalenten Anbindung der Detektionsmoleküle.
- Die Sekundärschicht enthält chemisch oder enzymatisch spaltbare Gruppen, um die quantitative Gewinnung von gebundenen Zielmolekülen oder Zielzellen zu vereinfachen.
- Die Sekundärschicht bildet eine Matrix, welche die Anbindung unspezifischer Zellen oder Wechselwirkungen mit Körperflüssigkeiten verhindert.
- An der Sekundärschicht sind Detektionsmoleküle kovalent angebunden.

Zur Verbesserung der Wirkung des Biodetektors und zur Vergrößerung der funktionalisierten Fläche, kann die funktionalisierte Fläche selbst und/oder der Untergrund, an welchem die funktionalisierte Fläche vorzusehen ist, eine Strukturierung aufweisen. Die Strukturierung kann Vorsprünge und/oder Vertiefungen umfassen, die beispielsweise zylindrisch, kugelabschnittsförmig, kegelförmig oder kegelstumpfförmig, pyramidenförmig oder pyramidenstumpfförmig ausgebildet sein können. Möglich ist allerdings auch eine Strukturierung in Form von Furchen, insbesondere Längs- und/oder Querfurchen. Vorzugsweise weist wenigstens eine der Innenwände des Flüssigkeitsentnahmeabschnitts und/oder des Leitungsabschnitts und/oder der Speichervorrichtung und/oder der Detektionsvorrichtung eine solche Strukturierung auf.

Weitere vorteilhafte Ausführungen der Erfindung ergeben sich durch Kombinationen der in den Ansprüchen genannten Merkmale oder Teilmerkmale.

### Kurze Beschreibung der Zeichnungen

- Fig. 1: zeigt eine Offenbarung wobei der Biodetektor als Spritze mit Kanüle ausgebildet ist.
- Fig. 2: zeigt eine Offenbarung, wobei der Biodetektor als Spritze mit Kanüle und zwischengeschaltetem Schlauch ausgebildet ist.
- Fig. 3: zeigt ein Ausführungsbeispiel der Erfindung, wobei der Biodetektor als Spritze mit Kanüle und zwischengeschaltetem Schlauch mit funktionalisiertem Chip ausgebildet ist.

### Detaillierte Beschreibung

Der Biodetektor 1 und dessen einzelne Bestandteile werden nachstehend mit Bezug auf die beiliegenden Zeichnungen im Detail beschrieben.

### Biodetektor

Der Biodetektor 1 dient der Anreicherung und Isolierung von Molekülen oder Zellen aus dem menschlichen Körper. Dazu ist der Biodetektor 1 derart ausgebildet, dass eine Flüssigkeit, insbesondere Blut, aus dem menschlichen Körper entnommen und in einem Innenraum 20, 30, 40, 50 des Biodetektors 1 aufgenommen werden kann. Eine funktionalisierte Fläche 21, 31, 41, die zumindest abschnittsweise mit Detektionsmolekülen besetzt ist, ist dem Innenraum 20, 30, 40, 50 des Biodetektors 1 zugewandt. Das bedeutet, dass die im Innenraum 20, 30, 40, 50 des Biodetektors 1 aufgenommene Flüssigkeit unmittelbar mit den Detektionsmolekülen in Kontakt gelangen kann, um dort isoliert bzw. angereichert zu werden. Vorzugsweise sind verschiedene funktionalisierte Flächen 21, 31, 41, 51, ggf. für verschiedene Zielmoleküle oder Zielzellen, in verschiedenen Abschnitten 2, 3, 4, 5 des Biodetektors 1 vorgesehen.

### Funktionalisierte Fläche

Die bevorzugten Detektionsmoleküle bzw. Rezeptoren oder Liganden auf der funktionalisierten Fläche sind zum Beispiel monoklonale Antikörper murinen Ursprungs, chimäre Antikörper, humanisierte Antikörper, Fragmente von Antikörpern oder Aminosäure-Strukturen bzw. Aminosäure-Sequenzen, Nukleinsäure-Strukturen bzw. Nukleinsäure-Sequenzen, Kohlehydratstrukturen oder synthetische Strukturen mit spezifischer Affinität zu Zelloberflächen oder Molekülen. Die Detektionsmoleküle können nicht-humanen Ursprungs, insbesondere tierischen Ursprungs sein. Natürlich kann die funktionalisierte Fläche auch mit verschiedenartigen Detektionsmolekülen besetzt werden.

### Anbindung der Detektionsmoleküle

Je nach dem Grundmaterial des entsprechenden Abschnitts 2, 3, 4, 5 des Biodetektors 1 können die Detektionsmoleküle unmittelbar oder mittelbar (über eine oder mehrere Zwischenschichten) an die funktionalisierte Fläche 21, 31, 41, 51 angebunden werden (Der Einfachheit halber wird nicht unterschieden zwischen einer "funktionalisierten Fläche", welche die Detektionsmoleküle bereits aufweist", und einer "zu funktionalisierenden Fläche", die durch Anbindung der Detektionsmoleküle funktionalisiert wird). Die funktionalisierte Fläche 21, 31, 41, 51 befindet sich wahlweise im Bereich des Flüssigkeitsentnahmeabschnitts 2 und/oder des Leitungsabschnitts 3 und/oder der Speichervorrichtung 4 und/oder der Detektionsvorrichtung 5.

Zur mittelbaren Anbindung der Detektionsmoleküle kann eine kovalente Sekundärschicht bestehend aus einem funktionalen, vorzugsweise biokompatiblen Polymer, bspw. Hydrogel, über nasschemische Verfahren auf den Untergrund aufgetragen werden. Dieses funktionale Polymer kann organische funktionelle Gruppen aufweisen, die in der Lage sind, Liganden oder Rezeptoren kovalent zu binden. Die Art der funktionellen Gruppen der Sekundärschicht richtet sich nach den Moleküleigenschaften der spezifischen Liganden und Rezeptoren. Zusätzlich können in diesem Polymer chemisch oder enzymatisch spaltbare Gruppen enthalten sein, um die quantitative Gewinnung von gebundenen Zielmolekülen oder Zielzellen zu vereinfachen. Falls erforderlich kann die Sekundärschicht eine Matrix bilden, welche die Anbindung unspezifischer Zellen oder Wechselwirkungen mit Körperflüssigkeiten verhindert.

Zur unmittelbaren Anbindung der Detektionsmoleküle an einem der Abschnitte 2, 3, 4, 5 des Biodetektors 1 sollte der Untergrund (zumindest der Teil, an welchem die funktionalisierte Fläche vorzusehen ist) zumindest eines der oben genannten Merkmale der Sekundärschicht umfassen. Zur Vergrößerung der funktionalisierten Fläche kann die funktionalisierte Fläche selbst und/oder der Untergrund, an welchem die funktionalisierte Fläche vorzusehen ist, eine Strukturierung aufweisen.

In einem Beispiel werden monoklonale Antikörper murinen Ursprungs als Detektionsmoleküle mittels Protein G kovalent und orientiert an der Innenwand des Flüssigkeitsentnahmeabschnitts 2 und/oder des Leitungsabschnitts 3 und/oder der Speichervorrichtung 4 und/oder der Detektionsvorrichtung 5 angebunden.

### Erstes Beispiel

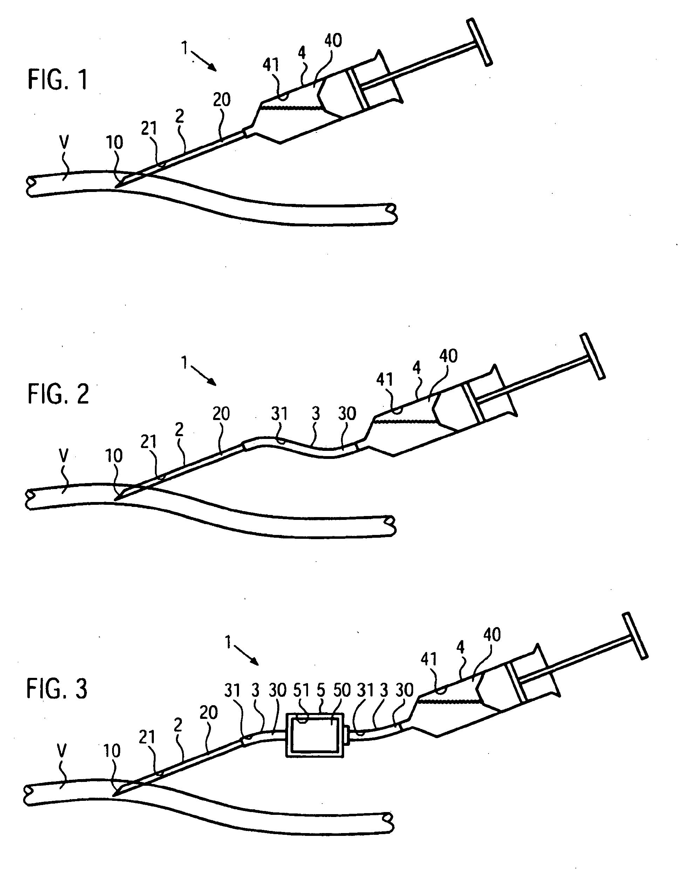

Im ersten Beispiel, das schematisch in Fig. 1 dargestellt ist, umfasst der Biodetektor 1 einen Flüssigkeitsentnahmeabschnitt 2, der lösbar mit einer Speichervorrichtung 4 verbunden ist. Die funktionalisierte Fläche befindet sich an einer Innenwand 21 des Flüssigkeitsentnahmeabschnitts 2 und/oder an der Innenwand 41 der Speichervorrichtung 4.

Der Flüssigkeitsentnahmeabschnitt 2 ist als Kanüle oder Hohlnadel aus einem biokompatiblem Material, insbesondere Edelstahl, Titan, Glasfaser, einem bioverträglichen Kunststoff oder einer Kombination aus diesen Substanzen ausgebildet. Das Aussehen bzw. die Form des Flüssigkeitsentnahmeabschnitts 2 kann variabel sein und ist abhängig von der Anwendung. Der Flüssigkeitsentnahmeabschnitt 2 ist bestimmungsgemäß dafür vorgesehen, die Haut und das Gewebe eines menschlichen Körpers zu durchschneiden bzw. zu durchstechen. Über eine Öffnung 10 am vorderen Ende kann eine Flüssigkeit aus dem menschlichen Körper in den Innenraum 20, 40 des Biodetektors 1 gelangen.

Die Speichervorrichtung 4 ist als Spritze ausgebildet und derart lösbar mit dem Flüssigkeitsentnahmeabschnitt 2 verbunden, dass der Innenraum 40 der Speichervorrichtung 4 mit der Öffnung 10 und mit dem Innenraum 20 des Flüssigkeitsentnahmeabschnitts 2 kommuniziert. Der Innenraum 40 der Speichervorrichtung 4 weist ein variables Volumen auf. Durch Vergrößerung des Innenraums 40 der Speichervorrichtung 4 wird über die kommunizierenden Innenräume 20, 40 ein Unterdruck erzeugt, so dass eine Flüssigkeit aus dem menschlichen Körper über eine Öffnung 10 in den Innenraum 20, 40 des Biodetektors 1 gelangt. Durch Verkleinerung des Innenraums 40 der Speichervorrichtung 4 kann die Flüssigkeit wieder aus dem Innenraum 20, 40 des Biodetektors 1 ausgestoßen werden.

### Zweites Beispiel

Das zweite Beispiel, das schematisch in Fig. 2 dargestellt ist, entspricht weitgehend dem ersten Beispiel. Abweichend vom ersten Beispiel ist ein Leitungsabschnitt 3 zwischen dem Flüssigkeitsentnahmeabschnitt 2 und der Speichervorrichtung 4 angeordnet. Die funktionalisierte Fläche befindet sich an der Innenwand 21 des Flüssigkeitsentnahmeabschnitts 2 und/oder an der Innenwand 31 des Leitungsabschnitts 3 und/oder an der Innenwand 41 der Speichervorrichtung 4.

Der Leitungsabschnitt 3 ist als flexibler Polymerschlauch, insbesondere als Polystyrenschlauch, ausgebildet und lösbar mit dem Flüssigkeitsentnahmeabschnitt 2 und der Speichervorrichtung 4 verbunden, so dass ein Innenraum 30 des Leitungsabschnitts 3 mit der Öffnung 10 sowie mit den Innenräumen 20, 40 des Flüssigkeitsentnahmeabschnitts 2 und der Speichervorrichtung 4 kommuniziert. Abweichend von der Darstellung in Fig. 2 kann der Leitungsabschnitt 3 eine Querschnittsänderung und/oder wenigstens eine Verzweigung mit offenen, kurzgeschlossenen und/oder verzweigten Zweigleitungen aufweisen.

### Ausführungsbeispiel

Das Ausführungsbeispiel, das schematisch in Fig. 3 dargestellt ist, entspricht weitgehend dem zweiten Beispiel. Abweichend vom zweiten Beispiel ist eine Detektionsvorrichtung 5 in den Leitungsabschnitt 3 derart integriert, dass ein Innenraum 50 der Detektionsvorrichtung 5 mit der Öffnung 10 und mit den Innenräumen 20, 30, 40 des Flüssigkeitsentnahmeabschnitts 2, des Leitungsabschnitts 3 und der Speichervorrichtung 4 kommuniziert. Die funktionalisierte Fläche befindet sich an der Innenwand 21 des Flüssigkeitsentnahmeabschnitts 2 und/oder an der Innenwand 31 des Leitungsabschnitts 3 und/oder an der Innenwand 41 der Speichervorrichtung 4 und/oder an der Innenwand 51 der Detektionsvorrichtung 5. Die Detektionsvorrichtung 5 ist als funktionalisierter Chip zur optischen oder computergestützten Erkennung und Bestimmung der Zielmoleküle und Zielzellen ausgebildet.

### Anwendung des Biodetektors

Der Flüssigkeitsentnahmeabschnitt 2 wird bestimmungsgemäß derart durch die Haut in einen menschlichen Körper eingeführt, dass sich die Öffnung 10 innerhalb des Körpers und das andere Ende des Flüssigkeitsentnahmeabschnitts 2 außerhalb des Körpers befinden. Vorzugsweise befindet sich die Öffnung 10 innerhalb einer Blutbahn V.

Durch Aufziehen der Spritze 4 und die damit verbundene Sogwirkung wird eine Flüssigkeit aus dem menschlichen Körper entnommen und über die Öffnung 10 in den Innenraum 20, 30, 40, 50 des Biodetektors 1 gefördert, so dass die Flüssigkeit mit der funktionalisierten Fläche 21, 31, 41, 51 in Kontakt gelangt und die in der Flüssigkeit enthaltenen Zielmoleküle und Zielzellen an der funktionalisierten Fläche 21, 31, 41, 51 angereichert werden können. In den Fig. 1, 2 und 3 ist der Innenraum 40 der Speichervorrichtung 4 jeweils teilweise mit der aus dem menschlichen Körper entnommenen Flüssigkeit befüllt.

### Anwendungsbeispiele

Der Biodetektor 1 eignet sich für die Gewinnung seltener Zellen aus Körperflüssigkeiten, insbesondere aus dem Blutkreislauf. Hierzu zählen folgende Anwendungsbeispiele:
- Gewinnung von embryonalen Zellen aus dem mütterlichen Blutkreislauf mit z.B. spezifischen Antikörperfragmenten (F(ab)-Fragmenten) und murinen monoklonalen Antikörpern (lgG), welche typische Zelloberflächenproteine von embryonalen Zellen, wie z.B. HLA-G erkennen können.
- Gewinnung von disseminierten Tumorzellen, insbesondere von hämatogen metastasierenden Tumoren z.B. mit dem humanisierten Antikörper Anti-EpCAM, welcher das für viele Krebszellen typische Zelloberflächenprotein EpCAM erkennt.

Der Biodetektor 1 eignet sich aber auch für die Gewinnung seltener Moleküle aus dem Blutkreislauf, insbesondere Tumormarkern oder Biomarkem.

Der Biodetektor 1 eignet sich ferner auch für die Elimination von Arzneimitteln aus dem Blutkreislauf. Hierzu zählen folgende Anwendungsbeispiele:
- Elimination von radioaktiven Tracern für Diagnostik und Therapie.
- Elimination von Magnetobeads bzw. supraparamagnetischen Partikeln mit gekoppelten Arzneimitteln.
- Elimination von Toxinen.

Diese Anwendungsbeispiele werden lediglich beispielhaft genannt und sind nicht abschließend zu verstehen.

### Vorteile der Erfindung

Durch den Biodetektor 1 kann eine bestimmte Menge einer Körperflüssigkeit, insbesondere Blut, aus dem menschlichen Körper entnommen und im Innenraum des Biodetektors aufgenommen sowie analysiert werden. Das mit der funktionalisierten Fläche in Kontakt gelangende Blut wird nicht in den Körper des Probanden zurückgeführt und ist nicht mehr Bestandteil des Blutkreislaufs. Somit ist die Verwendung von humanen Detektionsmolekülen nicht mehr erforderlich und sämtliche Biokompatibilitätstests können entfallen. Bspw. können Detektionsmoleküle tierischen Ursprungs, insbesondere monoklonale Antikörper murinen Ursprungs, verwendet werden, die mittels Protein G orientiert angebunden werden. Das Blut kann langsam durch die Kanüle 2 entnommen werden, so dass keine Probleme mehr mit den negativen Effekten der shear forces, die im strömenden Blut Zellbindung verhindern können, zu erwarten sind. Ferner kann das Blut nach der Entnahme mehrmals durch die Kanüle 2 gedrückt werden, so dass die entnommene Blutmenge damit zweimal für die Anbindung der Zielmoleküle und Zielzellen genutzt wird. Durch den modularen Aufbau des erfindungsgemäßen Biodetektors 1 kann die funktionalisierte Fläche beliebig groß gestaltet werden. Entsprechende Maßnahmen zur Vergrößerung der funktionalisierten Fläche können ergriffen werden, so dass bei einem vergleichsweise geringen Flüssigkeitsvolumen mehr Zellen aus der Körperflüssigkeit mit der funktionalisierten Fläche in Kontakt gelangen. Zu diesem Zweck können Zweigleitungen, feine Verästelungen (vgl. menschliche Lunge) und/oder Querschnittsänderungen vorgesehen werden. Im Inneren des Biodetektors 4-sind die funktionalisierte Fläche sowie die angereicherten Zielmoleküle und Zielzellen gut geschützt, wobei ein Abrieb durch das Einbringen des Biodetektors 1 in den Körper sowie durch anschließendes Herausnehmen verhindert werden kann. Dadurch ergeben sich weitere Vorteile bei der nachfolgenden Handhabung. Die detektierten bzw. isolierten Zellen und Moleküle können relativ einfach gespült und lysiert werden. Im Gegensatz zu alternativen Verfahren (Cell Search) muss die Körperflüssigkeit somit nicht an entsprechende Labors verschickt werden, um Zellen und Moleküle zu isolieren. Erfahrungen und Ausrüstungen für Umgang mit magnetic beads nicht erforderlich. Die eingesetzten Geräte bzw. Utensilien lassen sich problemlos handhaben und sind nicht ortsgebunden und überall einsetzbar.

## Patentansprüche

1. Biodetektor mit einer funktionalisierten Fläche zur Isolierung von Molekülen oder Zellen aus dem menschlichen Körper, wobei der Biodetektor derart ausgebildet ist, um eine Flüssigkeit aus dem menschlichen Körper zu entnehmen und in einem Innenraum des Biodetektors aufzunehmen, wobei die funktionalisierte Fläche dem Innenraum des Biodetektors zugewandt ist, wobei der Biodetektor einen Leitungsabschnitt aufweist sowie eine als Spritze ausgebildete Speichervorrichtung mit variablem Volumen, wobei der Innenraum der Speichervorrichtung mit dem Innenraum des Leitungsabschnitts kommunizieren kann, wobei sich die funktionalisierte Fläche an einer Innenwand des Leitungsabschnitts befindet, wobei der Leitungsabschnitt wenigstens eine Querschnittsänderung umfasst und die funktionalisierte Fläche an der Querschnittsänderung vorgesehen ist, wobei der Innenraum der Speichervorrichtung durch Vergrößerung des Speichervolumens mit der aus dem menschlichen Körper entnommenen Flüssigkeit befüllt werden kann.

2. Biodetektor nach Anspruch 1, **dadurch gekennzeichnet, dass** der Biodetektor einen Flüssigkeitsentnahmeabschnitt aufweist, der zumindest eine der folgenden Anforderungen erfüllt:
a. Der Flüssigkeitsentnahmeabschnitt umfasst eine Öffnung über welche die Flüssigkeit in den Innenraum des Biodetektors gelangt.
b. Ein Innenraum des Flüssigkeitsentnahmeabschnitts kommuniziert mit der Öffnung.
c. Die Öffnung ist an einem Ende des Flüssigkeitsentnahmeabschnitts ausgebildet.
d. Der Flüssigkeitsentnahmeabschnitt ist aus Kunststoff, Metall oder Glas, vorzugsweise Edelstahl, Titan oder Glasfaser, bevorzugt einer Kombination aus diesen Substanzen, ausgebildet.
e. Der Flüssigkeitsentnahmeabschnitt ist als Kanüle ausgebildet.
f. Der Flüssigkeitsentnahmeabschnitt umfasst einen Außendurchmesser von 0,25 bis 3,5 mm, vorzugsweise 0,5 bis 3,0 mm, weiter vorzugsweise 0,75 bis 2,5 mm, bevorzugt 1,0 mm bis 2,0 mm.
g. Die Öffnung umfasst einen Durchmesser von 0,2 bis 3,0 mm, vorzugsweise 0,25 bis 2,5 mm, weiter vorzugsweise 0,5 bis 2,0 mm, bevorzugt 0,75 mm bis 1,5 mm.
h. Die funktionalisierte Fläche befindet sich an einer Innenwand des Flüssigkeitsentnahmeabschnitts.

3. Biodetektor nach zumindest einem der vorangegangenen Ansprüche, **dadurch gekennzeichnet, dass** der Leitungsabschnitt zumindest eine der folgenden Anforderungen erfüllt:
a. Ein Innenraum des Leitungsabschnitts kommuniziert mit der Öffnung und/oder mit dem Innenraum des Flüssigkeitsentnahmeabschnitts.
b. Der Leitungsabschnitt ist mit dem Flüssigkeitsentnahmeabschnitt verbunden, vorzugsweise lösbar verbunden.
c. Der Leitungsabschnitt ist als flexibler Schlauch ausgebildet.
d. Der Leitungsabschnitt weist einen größeren Innendurchmesser und/oder Außendurchmesser auf als der Flüssigkeitsentnahmeabschnitt.
e. Der Leitungsabschnitt umfasst einen Innendurchmesser von 0,25 bis 3,5 mm, vorzugsweise 0,5 bis 3,0 mm, weiter vorzugsweise 0,75 bis 2,5 mm, bevorzugt 1,0 mm bis 2,0 mm.
f. Der Leitungsabschnitt umfasst wenigstens eine Verzweigung.
g. Der Leitungsabschnitt umfasst wenigstens eine offene Zweigleitung und/oder wenigstens eine kurzgeschlossene Zweigleitung und/oder wenigstens eine verzweigte Zweigleitung.
h. Der Leitungsabschnitt umfasst wenigstens drei Zweigleitungen, die sich in unterschiedlichen Ebenen erstrecken.
i. Der Leitungsabschnitt umfasst wenigstens eine Querschnittserweiterung und/oder wenigstens eine Querschnittsverringerung.
j. Der Leitungsabschnitt ist aus Kunststoff, vorzugsweise einem Polymer, bevorzugt Polystyren, ausgebildet.

4. Biodetektor nach zumindest einem der vorangegangenen Ansprüche, **dadurch gekennzeichnet, dass** die Speichervorrichtung zumindest eine der folgenden Anforderungen erfüllt:
a. Der Innenraum der Speichervorrichtung kommuniziert mit der Öffnung und/oder mit dem Innenraum des Flüssigkeitsentnahmeabschnitts.
b. Die Speichervorrichtung ist mit dem Leitungsabschnitt und/oder mit dem Flüssigkeitsentnahmeabschnitt verbunden, vorzugsweise lösbar verbunden.
c. Die funktionalisierte Fläche befindet sich an einer Innenwand der Speichervorrichtung.

5. Biodetektor nach zumindest einem der vorangegangenen Ansprüche, **dadurch gekennzeichnet, dass** der Biodetektor eine Detektionsvorrichtung aufweist, die zumindest eine der folgenden Anforderungen erfüllt:
a. Die Detektionsvorrichtung dient der optischen, vorzugsweise mikroskopisch gestützten Erkennung und/oder Bestimmung der Zielmoleküle oder Zielzellen.
b. Die Detektionsvorrichtung dient der computergestützten Erkennung und/oder Bestimmung der Zielmoleküle oder Zielzellen.
c. Die Detektionsvorrichtung ist in den Flüssigkeitsentnahmeabschnitt und/oder in den Leitungsabschnitt und/oder in die Speichervorrichtung integriert.
d. Der Innenraum der Detektionsvorrichtung kommuniziert mit der Öffnung und/oder mit dem Innenraum des Flüssigkeitsentnahmeabschnitts und/oder dem Innenraum des Leitungsabschnitts und/oder mit dem Innenraum der Speichervorrichtung.
e. Die Detektionsvorrichtung ist mit dem Flüssigkeitsentnahmeabschnitt und/oder mit dem Leitungsabschnitt und/oder mit der Speichervorrichtung verbunden, vorzugsweise lösbar verbunden.
f. Die funktionalisierte Fläche befindet sich an einer Innenwand der Detektionsvorrichtung.

6. Biodetektor nach zumindest einem der vorangegangenen Ansprüche, **dadurch gekennzeichnet, dass** der Biodetektor eine Sekundärschicht aufweist, die zumindest eine der folgenden Anforderungen erfüllt:
a. Die Sekundärschicht ist als Polymerschicht, vorzugsweise als Hydrogelschicht ausgebildet.

7. Biodetektor nach zumindest einem der vorangegangenen Ansprüche, **dadurch gekennzeichnet, dass** die funktionalisierte Fläche eine Strukturierung aufweist, wobei die Strukturierung vorzugsweise Vorsprünge und/oder Vertiefungen umfasst, die bevorzugt zylindrisch, kugelabschnittsförmig, kegelförmig oder kegelstumpfförmig, pyramidenförmig oder pyramidenstumpfförmig, oder als Stege oder Furchen ausgebildet sind.

## Claims

1. Biodetector comprising a functionalised surface for isolating molecules or cells from the human body, wherein the biodetector is configured to remove a fluid from the human body and to absorb it into an interior of the biodetector, wherein the functionalised surface faces the interior of the biodetector, wherein the biodetector comprises a line portion and a storage device in the form of a syringe having a variable volume, wherein the interior of the storage device can communicate with the interior of the line portion, wherein the functionalised surface is located on an inner wall of the line portion, wherein the line portion has at least one change in cross section, and the functionalised surface is provided on said change in cross section, wherein the interior of the storage device can be filled with the fluid removed from the human body by increasing the storage volume.

2. Biodetector according to claim 1, **characterised in that** the biodetector has a fluid removal portion, which meets at least one of the following requirements:
a. The fluid removal portion comprises an opening through which the fluid reaches the interior of the biodetector.
b. An interior of the fluid removal portion communicates with the opening.
c. The opening is formed at one end of the fluid removal portion.
d. The fluid removal portion is made of plastics material, metal or glass, preferably stainless steel, titanium, or fibreglass, preferably a combination of these substances.
e. The fluid removal portion is in the form of a cannula.
f. The fluid removal portion has an outer diameter of from 0.25 to 3.5 mm, preferably from 0.5 to 3.0 mm, more preferably from 0.75 to 2.5 mm, preferably from 1.0 mm to 2.0 mm.
g. The opening has a diameter of from 0.2 to 3.0 mm, preferably from 0.25 to 2.5 mm, more preferably from 0.5 to 2.0 mm, preferably from 0.75 mm to 1.5 mm.
h. The functionalised surface is located on an inner wall of the fluid removal portion.

3. Biodetector according to at least one of the preceding claims, **characterised in that** the line portion meets at least one of the following requirements:
a. An interior of the line portion communicates with the opening and/or with the interior of the fluid removal portion.
b. The line portion is connected, preferably detachably connected, to the fluid removal portion.
c. The line portion is in the form of a flexible tube.
d. The line portion has a larger inner diameter and/or outer diameter than the fluid removal portion.
e. The line portion has an inner diameter of from 0.25 to 3.5 mm, preferably from 0.5 to 3.0 mm, more preferably from 0.75 to 2.5 mm, preferably from 1.0 mm to 2.0 mm.
f. The line portion comprises at least one branch.
g. The line portion comprises at least one open branch line and/or at least one bypassed branch line and/or at least one branched branch line.
h. The line portion comprises at least three branch lines, which extend in different planes.
i. The line portion comprises at least one enlargement of the cross section and/or at least one reduction of the cross section.
j. The line portion is made of plastics material, preferably a polymer, preferably polystyrene.

4. Biodetector according to at least one of the preceding claims, **characterised in that** the storage device meets at least one of the following requirements:
a. The interior of the storage device communicates with the opening and/or with the interior of the fluid removal portion.
b. The storage device is connected, preferably detachably connected, to the line portion and/or to the fluid removal portion.
c. The functionalised surface is located on an inner wall of the storage device.

5. Biodetector according to at least one of the preceding claims, **characterised in that** the biodetector has a detection device which meets at least one of the following requirements:
a. The detection device provides optical, preferably microscope-assisted detection and/or identification of the target molecules or target cells.
b. The detection device provides computer-aided detection and/or identification of the target molecules or target cells.
c. The detection device is integrated in the fluid removal portion and/or the line portion and/or the storage device.
d. The interior of the detection device communicates with the opening and/or with the interior of the fluid removal portion and/or the interior of the line portion and/or with the interior of the storage device.
e. The detection device is connected, preferably detachably connected, to the fluid removal portion and/or to the line portion and/or to the storage device.
f. The functionalised surface is located on an inner wall of the detection device.

6. Biodetector according to at least one of the preceding claims, **characterised in that** the biodetector has a secondary layer which meets at least one of the following requirements:
a. The secondary layer is in the form of a polymer layer, preferably of a hydrogel layer.

7. Biodetector according to at least one of the preceding claims, **characterised in that** the functionalised surface is textured, the texture preferably comprising projections and/or recesses which are preferably in the form of cylinders, spherical segments, cones or truncated cones, pyramids or truncated pyramids, or of ridges or grooves.

## Revendications

1. Biodétecteur avec une surface fonctionnalisée destinée à isoler des molécules ou des cellules du corps humain, dans lequel le biodétecteur est constitué de manière à prélever un fluide du corps humain et à le recevoir dans un espace interne du biodétecteur, dans lequel la surface fonctionnalisée fait face à l'espace interne du biodétecteur, dans lequel le biodétecteur comporte une section de conduite ainsi qu'un dispositif de stockage à volume variable constitué sous forme d'une seringue, dans lequel l'espace interne du dispositif de stockage peut communiquer avec l'espace interne de la section de conduite, dans lequel la surface fonctionnalisée sur trouve sur une paroi interne de la section de conduite, dans lequel la section de conduite comporte au moins un changement de section transversale et la surface fonctionnalisée est pourvue au changement de section transversale, dans lequel l'espace interne du dispositif de stockage peut être rempli avec le fluide prélevé hors du corps humain par une augmentation du volume de stockage.

2. Biodétecteur selon la revendication 1, **caractérisé en ce que** le biodétecteur comporte une section de prélèvement de fluide qui répond à au moins l'une des exigences suivantes :
a. la section de prélèvement de fluide comporte une ouverture à travers laquelle le fluide parvient dans l'espace interne du biodétecteur ;
b. un espace interne de la section de prélèvement de fluide communique avec l'ouverture ;
c. l'ouverture est constituée à une extrémité de la section de prélèvement de fluide ;
d. la section de prélèvement de fluide est constituée en plastique, en métal ou en verre, préférablement en acier inoxydable, en titane ou en fibre de verre, et préférablement encore en une combinaison de ces matériaux ;
e. la section de prélèvement de fluide est conformée comme une canule ;
f. la section de prélèvement de fluide présente un diamètre externe de 0,25 mm à 3,5 mm, préférablement de 0,5 mm à 3,0 mm, préférablement encore de 0,75 mm à 2,5 mm, et préférablement encore de 1,0 mm à 2,0 mm ;
g. l'ouverture présente un diamètre de 0,2 mm à 3,0 mm, préférablement 0,25 mm à 2,5 mm, préférablement encore de 0,5 mm à 2,0 mm, et préférablement encore de 0,75 mm à 1,5 mm ;
h. la surface fonctionnalisée se trouve sur une paroi interne de la section de prélèvement de fluide.

3. Biodétecteur selon au moins l'une des revendications précédentes, **caractérisé en ce que** la section de conduite répond à au moins l'une des exigences suivantes :
a. un espace interne de la section de conduite communique avec l'ouverture et/ou avec l'espace interne de la section de prélèvement de fluide ;
b. la section de conduite est connectée à la section de prélèvement de fluide, préférablement de manière détachable ;
c. la section de conduite est constituée comme un tuyau flexible ;
d. la section de conduite présente un diamètre interne et/ou un diamètre externe supérieur à celui de la section de prélèvement de fluide ;
e. la section de conduite présente un diamètre interne de 0,25 mm à 3,5 mm, préférablement 0,5 mm à 3,0 mm, préférablement encore de 0,75 mm à 2,5 mm, et préférablement encore de 1,0 mm à 2,0 mm ;
f. la section de conduite comporte au moins une bifurcation ;
g. la section de conduite comporte au moins une conduite de dérivation ouverte et/ou au moins une conduite de dérivation court-circuitée et/ou au moins une conduite de dérivation ramifiée ;
h. la section de conduite comporte au moins trois conduites de dérivation qui s'étendent dans différents plans ;
i. la section de conduite comporte au moins une augmentation de section transversale et/ou au moins une diminution de section transversale ;
j. la section de conduite est constituée en plastique, préférablement en polymère, et préférablement encore en polystyrène.

4. Biodétecteur selon au moins l'une des revendications précédentes, **caractérisé en ce que** le dispositif de stockage répond à au moins l'une des exigences suivantes :
a. l'espace interne du dispositif de stockage communique avec l'ouverture et/ou avec l'espace interne de la section de prélèvement de fluide ;
b. le dispositif de stockage est connecté à la section de conduite et/ou à la section de prélèvement de fluide, préférablement de manière détachable ;
c. la surface fonctionnalisée se trouve sur une paroi interne du dispositif de stockage.

5. Biodétecteur selon au moins l'une des revendications précédentes, **caractérisé en ce que** le biodétecteur comporte un dispositif de détection qui répond à au moins l'une des exigences suivantes :
a. le dispositif de détection sert à une identification et/ou à une détermination à support optique, préférablement microscopique, des molécules ou cellules cibles ;
b. le dispositif de détection sert à une identification et/ou à une détermination à support informatique des molécules ou cellules cibles ;
c. le dispositif de détection est intégré dans la section de prélèvement de fluide et/ou dans la section de conduite et/ou dans le dispositif de stockage ;
d. l'espace interne du dispositif de détection communique avec l'ouverture et/ou avec l'espace interne de la section de prélèvement de fluide et/ou avec l'espace interne de la section de conduite et/ou avec l'espace interne du dispositif de stockage ;
e. le dispositif de détection est connecté à la section de prélèvement de fluide et/ou à la section de conduite et/ou au dispositif de stockage, préférablement de manière détachable ;
f. la surface fonctionnalisée se trouve sur une paroi interne du dispositif de détection.

6. Biodétecteur selon au moins l'une des revendications précédentes, **caractérisé en ce que** le biodétecteur comporte une couche secondaire qui répond à au moins l'une des exigences suivantes :
a. la couche secondaire est constituée comme une couche de polymère, préférablement comme une couche d'hydrogel.

7. Biodétecteur selon au moins l'une des revendications précédentes, **caractérisé en ce que** la surface fonctionnalisée comporte une structuration, dans lequel la structuration comporte préférablement des élévations et/ou des abaissements, qui sont préférablement cylindriques, à section sphérique, coniques ou tronconiques, pyramidaux ou en tronc de pyramide, ou constitués comme des nervures ou des rainures.
